# EUROPÄISCHE PATENTSCHRIFT

(11) **EP 0 543 309 B1**
(45) Veröffentlichungstag und Bekanntmachung des Hinweises auf die Patenterteilung: **02.11.1995**
(21) Anmeldenummer: 92119556.6
(22) Anmeldetag: 16.11.1992
(51) Int. Cl.: A61F 2/00, A61M 25/00

(54) **Vorrichtung zur Behandlung der Harninkontinenz des Mannes**
Device for the treatment of male urinary incontinence
Dispositif pour traiter l'incontinence urinaire masculine

(30) Priorität: 19.11.1991 DE 9114435 U
(43) Veröffentlichungstag der Anmeldung: 26.05.1993
(73) Patentinhaber: Engel, Konrad Dr.med., D-83674 Gaissach (DE)
(72) Erfinder: Engel, Konrad Dr.med., D-83674 Gaissach (DE)

(56) Entgegenhaltungen:
- EP-A- 0 265 207
- EP-A- 0 328 332
- US-A- 4 932 938
- US-A- 4 946 449

## Beschreibung

Die Erfindung betrifft eine Vorrichtung zur Behandlung der Harninkontinenz des Mannes mit den Merkmalen gemäß dem Oberbegriff des Anspruches 1.

Unter Harninkontinenz oder Streßharninkontinenz versteht man den unkontrollierten Verlust von Urin aus Blase und Harnröhre. Ursache ist eine Schädigung des Verschlußmechanismus der Harnblase, meist als Folge einer Prostataoperation, einer Infiltration des Schließmuskels durch ein Prostatakarzinom oder einer Nervenläsion, z.B. nach einer Dickdarmoperation. Betroffen sind in der Regel Männer höheren Alters.

Zur Behandlung und Behebung der Harninkontinenz sind bereits vielfältige Maßnahmen bekannt, die jedoch durchwegs nicht frei von Nachteilen sind. So werden Operationen ohne künstliche Implantate ausgeführt, meist unter Verlagerung von Beckenbodenmuskulatur, die die Funktion des Schließmuskels zu übernehmen hat. Eine derartige Operation ist aufwendig und vor allem für den Patienten belastend.

Weiterhin ist auch ein künstlicher Blasenschließmuskel bekannt (AMS 800 der Firma American Medical Systems), der ebenfalls operativ implantiert werden muß. Neben dem Nachteil einer Belastung für den Patienten durch die Operation ist dieser künstliche Blasenschließmuskel sehr teuer.

Eine bekannte Vorrichtung zur Behebung der Harninkontinenz besteht aus einer sog. Penisklemme oder Inkontinenz-Manschette, wobei letztere einen aufblasbaren Ballon enthält, durch den die Harnröhre von außen her abgeklemmt werden kann. Nachteilig an dieser bekannten Vorrichtung ist, daß die Klemme je nach Durchblutungszustand des Penis mehr oder weniger fest sitzt und daher bei Bewegungen leicht abgestreift werden kann und damit unwirksam ist. Um dies zu vermeiden, wird die Klemme häufig, z.B. durch stärkeres Aufblasen des genannten Ballons, gespannt, was wiederum zu Durchblutungsstörungen führt.

Bekannte Flüssigkeit absorbierende Windeleinlagen haben den Nachteil, daß urinbenetzte Hautregionen entzündlich gereizt werden und damit außerdem eine Geruchsbelästigung einhergehen kann, die bis hin zu sozialer Isolierung führt.

Weiterhin sind auch Kondom-Urinale mit Dauerablauf in einen Urinauffangbeutel bekannt, die kondomartig über den Penis gestülpt werden. Auch diese halten, ähnlich wie die vorstehend genannten Penisklemmen, nicht völlig sicher und die Unterbringung des Urinauffangbeutels im Hosenbein od.dgl. ist ausgesprochen lästig.

Schließlich ist auch eine Vorrichtung der im Oberbegriff des Anspruches 1 angegebenen Art bekannt, mit der grundsätzlich eine Reihe der vorstehend aufgezählten Nachteile vermieden werden kann (DE-OS 40 14 369 = US-A-4 932 938). Diese bekannte Vorrichtung besteht im wesentlichen aus einem in die Harnröhre einführbaren Katheter, der an seinem proximalen Endabschnitt einen mit einem Fluid (z.B. Wasser) füllbaren und dadurch erweiterbaren Ballon aufweist. Dieser Ballon dichtet die Harnblase am Eintritt in die Harnröhre ab und sichert den Katheter gegen ein ungewolltes Herausziehen. Distal von dem Ballon ist ein durch einen weiteren Kanal mit Fluid füllbarer zweiter Ballon angeordnet, der im eingesetzten Zustand des Katheters außerhalb des Blasenschließmuskels liegt und auf diese Weise ein ungewolltes Verschieben des Katheters in das Blaseninnere verhindert. Die Länge des Katheters ist derart bemessen, daß sein distales Ende im eingesetzten Zustand vollständig im Penis aufgenommen ist, und in dem distalen Endabschnitt ist ein Ventil angeordnet, das von außerhalb durch die Wandung der Harnröhre hindurch tastbar ist. Das Ventil ist beispielsweise ein Schnabel- oder Lippenventil, dessen normalerweise geschlossener Zustand sich durch Druck zwischen zwei Fingern in den geöffneten Zustand überführen lässt, so daß die Miktion stattfinden kann.

Die beiden Kanäle, die jeweils mit den genannten Ballonen in Verbindung stehen, erstrecken sich bis zu dem das Ventil aufnehmenden Endabschnitt des Katheters und sind in einem gewissen Abstand vom distalen Ende des Katheters durch eine mittels einer spitzen Kanüle durchstechbaren Wand verschlossen. Zum Einsetzen der Vorrichtung in die Harnröhre ist ein Drainage- und Aufblaselement vorgesehen, das stirnseitig ein Paar von Füllnadeln sowie eine Kanalverlängerung aus rostfreiem Stahl besitzt und in das distale Ende des Katheters eingesteckt werden kann. Beim Einstecken dringen die Füllnadeln in die stirnseitigen Mündungen der zu den Ballonen führenden Kanäle ein und durchstechen dabei nach einer bestimmten Eindringtiefe die genannten Wände, welche die Kanäle verschließen. Durch am distalen Ende des Drainage- und Aufblaselements vorgesehene Spritzenaufnahmen ist es möglich, über die so hergestellte Verbindung die Ballone mit einem Fluid zu füllen, nachdem der Katheter in die Harnröhre eingeführt und die Ballone richtig plaziert wurden. Nach dem Herausziehen des Drainage- und Aufblaselements und damit der Füllnadeln aus den zu den Ballonen führenden Kanälen schließen die Wandungen die Kanäle wieder dicht ab, so daß die Füllung der Ballone bestehen bleibt und der Katheter in der Harnröhre gehalten ist.

Zum erneuten Entfernen des Katheters, das in gewissen Zeitabständen erfolgen muß, ist es notwendig, das Doppelballon-System zu entleeren. Hierzu müssen die genannten Füllnadeln (oder andere spitze Kanülen) erneut in die Kanäle eingeführt und die Wandungen durchstochen werden, um eine Verbindung nach außen herzustellen. Dieser Vorgang erweist sich als außerordentlich schwierig und nur durchführbar, wenn das distale Ende des Katheters nahe an der Harnröhrenmündung angeordnet ist, weil sich andernfalls die Füllnadeln bzw. Kanülen nicht in die Kanäle so einführen lassen, daß ohne ein Hängenbleiben in den Kanalinnenwandungen oder gar eine Verletzung des Patienten ein sicheres Durchstechen der Wandungen gewährleistet ist. Auch bereitet es infolge der notwendigen Nachgiebigkeit des Katheters Schwierigkeiten, den für das Durchstechen notwendigen Druck auf den im Inneren der Harnröhre befindlichen Katheter auszuüben, ohne daß dieser ausweicht und dadurch die Spitzen der Füllnadeln oder Kanülen von der richtigen Stelle abgelenkt werden.

Die aus dem vorstehenden Grund notwendige Längenbemessung des Katheters so, daß dessen distales Ende relativ nahe an der Harnröhrenmündung liegt, hat den gravierenden Nachteil aller bekannten Dauerkatheter zur Folge, der darin besteht, daß eine Infektion der Harnröhre und der Harnblase mit daraus resultierender chronischer Entzündung infolge von längs dem Katheter aufsteigenden Keimen in der Praxis nicht vermieden werden kann. Denn die kurze Distanz zwischen dem Harnröhrenende und dem distalen Ende des Katheters kann durch Keime leicht überbrückt werden.

Hinzu kommt, daß ebenfalls infolge der verhältnismässig kurz vor der Harnröhrenmündung gelegenen Anordnung des distalen Katheterendes der Katheter den Sekretabfluß aus den etwa in der Harnröhrenmitte gelegenen Drüsen behindert und sogar staut, so daß Verkrustungen am distalen Katheterende auftreten können. Diese begünstigen die Keimausbreitung beträchtlich.

Der Erfindung liegt daher die Aufgabe zugrunde, eine Vorrichtung zur Behandlung und Behebung der Harninkontinenz der vorstehend beschriebenen Art so auszubilden, daß sie bei Komplikationen sofort und leicht ausgewechselt werden kann und insbesondere eine Infektionsgefahr ausschließt.

Erfindungsgemäß wird diese Aufgabe gelöst durch die Ausgestaltung der Vorrichtung gemäß dem Kennzeichen des Anspruches 1.

Bei der erfindungsgemässen Vorrichtung handelt es sich um einen in der Harnröhre so weit versenkbaren Inkontinenz-Katheter, daß dieser - weil keinerlei Verbindung mit der äußeren Körperoberfläche besteht - ein Aufsteigen von Keimen ausschließt. Die zur Entfernung des Inkontinenz-Katheters notwendige Entleerung des Doppelballon-Systems kann leicht durch Perforation der die distale Mündung des Füllkanals verschließendenMembran durchgeführt werden, weil diese Membran infolge ihrer stirnseitigen Anordnung leicht zu finden und zu ritzen ist. Infolgedessen kann die Länge des Inkontinenz-Katheters so bemessen werden, daß sein distales Ende weit entfernt von der Harnröhrenmündung angeordnet ist und proximal von den in der Harnröhre befindlichen Sekretdrüsen liegt. Aufgrund dieser grösseren Distanz zur Harnröhrenmündung und insbesondere aufgrund des Wegfalls der Behinderung des Sekretflusses kann das Aufsteigen von Keimen in der Harnröhre und längs des Katheters verhindert werden.

Die übrigen Vorteile des Inkontinenz-Katheters wie Wegfall von Durchblutungsstörungen des Penis sowie von Reizungen durch urinbenetzte Hautregionen oder Geruchsbelästigungen und völlige Unsichtbarkeit des Katheters im Benutzungszustand bleiben erhalten. Auch das Einsetzen des erfindungsgemässen Katheters ist trotz des grösseren Abstandes seines distalen Endes und der Harnröhrenmündung ambulant und kostengünstig möglich und der Katheter ist relativ einfach aufgebaut, so daß er billig und als Einmalartikel herstellbar ist. Zur Herstellung bedarf es im wesentlichen keiner bisher ungebräuchlichen technischen Verfahren. Die infrage kommenden Werkstoffe sind physiologisch verträgliche Kunststoffe, insbesondere Silikonkautschuk-Verbindungen mit guter Schleimhautverträglichkeit, die ein Belassen des Katheters in den Harnwegen für einen Zeitraum zwischen vier Wochen und sechs Monaten ermöglichen.

Ein Ausführungsbeispiel der Erfindung ist nachfolgend anhand der beiliegenden Zeichnungen näher erläutert. In den Zeichnungen zeigen:
Fig. 1 eine schematische Darstellung des erfindungsgemässen Inkontinenz-Katheters, in der zur Verdeutlichung das distale Ende maßstäblich vergrössert dargestellt ist;
Fig. 2 eine schematische Darstellung, die den eingesetzten Zustand des Inkontinenz-Katheters zeigt, und
Fig. 3 eine schematische, im Maßstab vergrösserte Stirnansicht des distalen Katheterendes im eingesetzten Zustand, welche die Art der Betätigung des Ventils veranschaulicht.

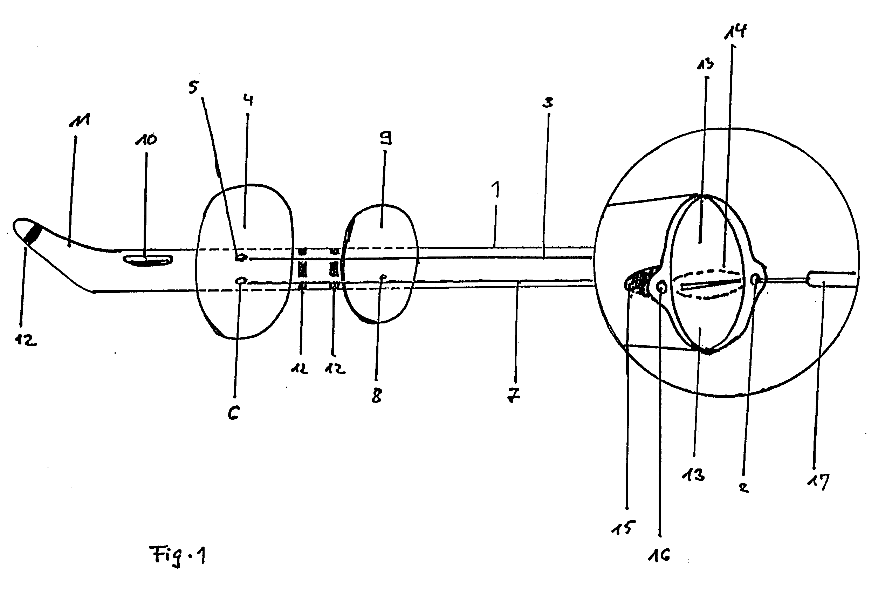

Gemäß Fig. 1 besteht die erfindungsgemässe Vorrichtung aus einem schlauch- oder rohrförmigen Katheter 1, z.B. aus Silikonkautschuk, dessen Länge so bemessen ist, daß er im eingesetzten Zustand mit seinem distalen - in Fig. 1 rechten - Ende innerhalb der Harnröhre liegt. In der Nähe des proximalen Endes 11 ist mit der Außenseite des Katheters 1 ein erster Ballon 4 fest verbunden. In einem gewissen Abstand distal davon ist an der Außenseite des Katheters 1 ein zweiter Ballon 9 fixiert. Vom distalen Ende des Katheters 1 aus verläuft in dessen Wandung ein Kanal 3, der über eine in der Katheterwand vorgesehene Öffnung 5 in das Innere des proximalen Ballons 4 mündet. Neben der Öffnung 5 ist in der Außenfläche des Katheters 1 eine weitere Öffnung 6 ausgebildet, von der aus ein Verbindungskanal 7, ebenfalls in der Katheterwandung, zum distalen Ende des Katheters 1 verläuft. Der Verbindungskanal 7 steht jedoch über eine Öffnung 8 mit dem Inneren des distalen Ballons 9 in Verbindung. Die Öffnung 8 hat einen deutlich kleineren Querschnitt als die beiden Öffnungen 5 und 6, um auf das dadurch in das Innere des Ballons 9 eintretende Fluid eine Drosselwirkung auszuüben.

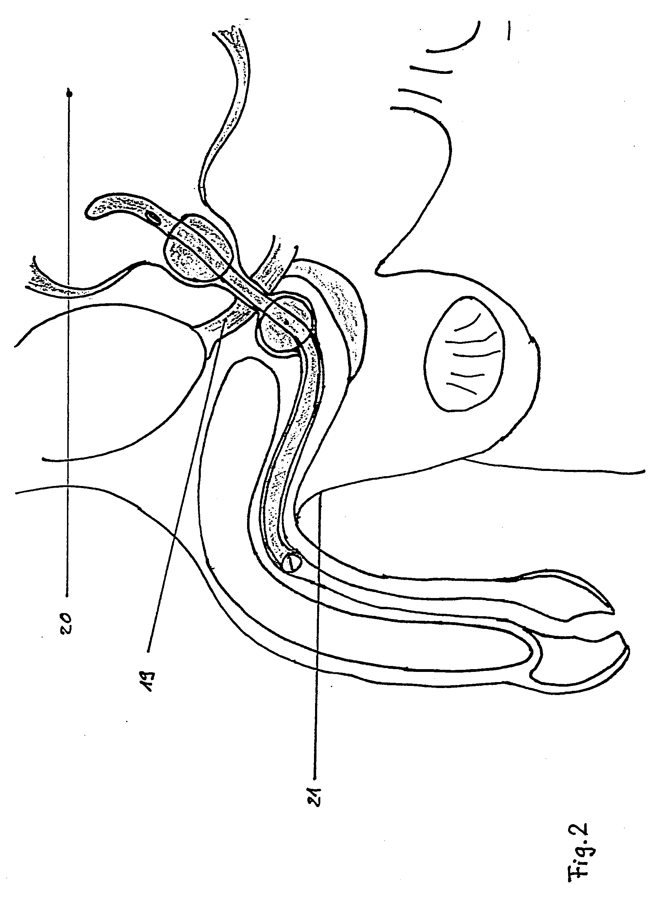

Die proximale, leicht gebogene und konische Katheterspitze 11 ragt im eingesetzten Zustand in das Lumen der Harnblase 20 (Fig. 2). In einem geringen Abstand davon weist die Katheterwandung einen Durchbruch 10 auf, durch welche das Harnblasenlumen mit dem Inneren des Katheters verbunden ist.

Das distale Katheterende enthält ein Lippenventil, welches im wesentlichen aus zwei halbmondförmigen elastischen Lippen 13 besteht, deren sich berührende Ränder wasserdicht schließen. In die Lippen 13 ist ein etwa elliptischer, geschlossener und federelastischer Bügel 14 eingebettet, welcher die Dichtränder umschließt und deren gegenseitigen Dichtkontakt unterstützt. In dem vorliegenden Ausführungsbeispiel sind die Lippen 13 in einem federelastischen Ring gehalten, der mit dem distalen Ende des Katheters 1 fest verbunden, z.B. damit verschweißt ist. Dieser Ring weist einander diametral gegenüberliegende Ohren auf, die mit entsprechenden seitlichen Vorsprüngen oder Randleisten 15 an der Außenwand des Katheters 1 korrespondieren und mit deren Stirnseite verbunden sind. In den Randleisten 15 münden jeweils der Kanal 3 bzw. der Verbindungskanal 7, wobei in der Mündung 2 des Kanals 3 ein normalerweise geschlossenes gummielastisches Ventil angeordnet ist, während die Mündung 16 des Verbindungskanals 7 durch eine elastische Membran verschlossen ist.

An der proximalen Katheterspitze 11 sowie zwischen den beiden Ballonen 4 und 9 sind röntgendichte Markierungen 12 vorgesehen, welche die Lagekontrolle des Katheters beim Einsetzen erleichtern.

Das Material des Katheters 1, dessen Wandstärke bzw. Biegeweichheit sowie die Anordnung von Kanälen in der Katheterwandung sind von bekannten Dauerkathetern her bekannt und bedürfen daher an dieser Stelle keiner ins einzelne gehenden Erläuterung. Anstelle einer Führung der Kanäle 3 und 7 unmittelbar in der Katheterwandung ist es auch möglich, diese Kanäle im Inneren des Katheters an der Katheterinnenwand entlang zu führen.

Die erfindungsgemässe Vorrichtung wird auf folgende Weise eingesetzt:
In die distale Mündung 2 des Kanals 3, durch das dort vorgesehene gummielastische Ventil hindurch, wird eine lange Kanüle 17 mit einer stumpfen Spitze eingeführt. Mittels der Kanüle 17 kann so die Vorrichtung in die Harnröhre geschoben werden. Hat das proximale Ende 11 des Katheters 1 die Harnblase 20 erreicht und ragt in das Blasenlumen, so wird über die Kanüle 17 ein Fluid, in der Regel Wasser, in den Kanal 3 eingedrückt. Das Wasser gelangt über die Öffnung 5 in das Innere des Ballons 4, füllt diesen und weitet ihn dabei auf. Zugleich gelangt Wasser auch über die Öffnung 6 in den Verbindungskanal 7 und über dessen Öffnung 8 in das Innere des distalen Ballons 9. Da der Querschnitt dieser Öffnung 8 jedoch deutlicher geringer ist, wird der Zufluß von Wasser in den distalen Ballon 9 merklich gedrosselt, so daß dieser sich erheblich langsamer als der proximale Ballon 4 füllt. Während der so stattfindenden zeitlichen Verzögerung wird der Katheter 1 durch vorsichtigen Zug an der Kanüle 17 bis zum fühlbaren Widerstand an dem Schließmuskel 19 (Fig. 2) zurückgezogen. Nach der vollständigen Füllung auch des distalen Ballons 9 sorgt das Doppelballonsystem sowohl für die Abdichtung der proximalen Harnröhre als auch für die Lagestabilität und Lagesicherung der Vorrichtung. Im vollständig gefüllten Zustand des Doppelballonsystems enthält auch der Verbindungskanal 7 bis zu seiner distalen Mündung 16 hin Wasser, unter dessen leichtem Druck sich die dort vorgesehene Membran auswölbt.

Im eingesetzten Zustand der Vorrichtung reicht das distale Katheterende, in welchem das Lippenventil 13 angeordnet ist, etwa bis zum distalen Ansatz des Skrotums 21 am Penis und ist dort, insbesondere aufgrund der daran vorgesehenen Randleisten 15, zwischen Daumen und Zeigefinger tastbar.

Soll die Vorrichtung wieder entfernt werden, so wird die Membran in der distalen Mündung 16 des Verbindungskanals 7 unter Sicht mit dem Urethroskop mit einer Punktionsnadel oder einer Biopsie-Zange perforiert. Daraufhin können sich die Ballone 4 und 9 rasch entleeren und die Vorrichtung kann leicht aus der Harnröhre herausgezogen werden. Prinzipiell wäre eine Entleerung der Ballone 4 und 9 auch über die distale Mündung 2 des Kanals 3 möglich, jedoch ist die Intubation des Kanals 3 mit einer Kanüle im eingesetzten Zustand der Vorrichtung sehr schwierig, so daß durch die Verlängerung des Verbindungskanals 7 bis zum distalen Katheterende hin diesbezüglich eine Erleichterung geschaffen ist.

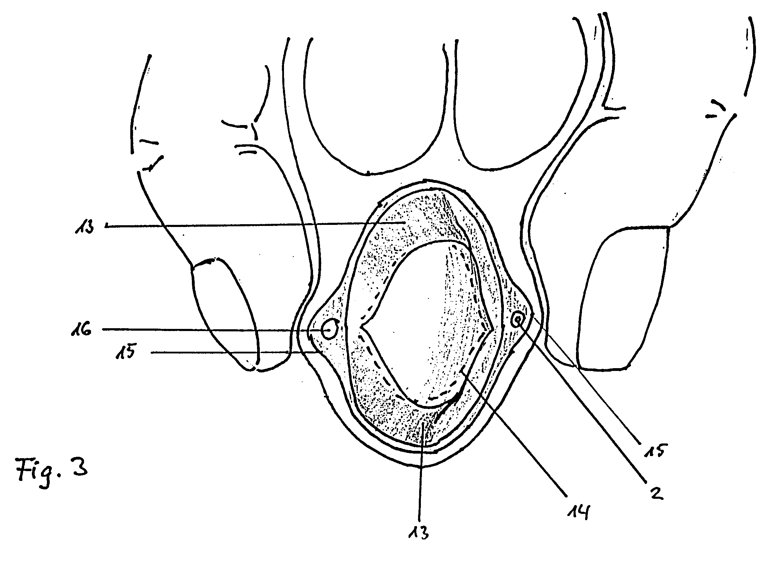

Der schematische Querschnitt des männlichen Gliedes in Höhe des Skrotalansatzes gemäß Fig. 3 veranschaulicht die Wirkungsweise der erfindungsgemässen Vorrichtung bei deren Benutzung durch den Patienten. Durch gleichzeitigen seitlichen Druck von Daumen und Zeigefinger auf die durch die Harnröhrenwand hindurch tastbaren Randleisten 15 kann der federelastische Ring, in welchem die Ventillippen 13 gehalten sind, und der die Dichtränder der Lippen 13 umgebende Bügel 14 radial und in Längsrichtung der Dichtränder eingedrückt werden, so daß diese sich dadurch fischmaulartig öffnen. Ist ein federelastischer Ring nicht vorhanden, weil die Ventillippen 13 unmittelbar mit der Innenwand des Katheters 1 verbunden sind, so wird der Druck direkt auf den elastischen Bügel 14 ausgeübt. Bei geöffneten Dichträndern erfolgt die Miktion. Durch Loslassen der Randleisten 15 schließt sich das Katheterventil wieder selbsttätig infolge der elastischen Rückstellkraft der Ventillippen 13, des Bügels 14 und ggf. des federelastischen Ringes.

Im Rahmen der vorliegenden Erfindung kann von Einzelheiten des vorstehend geschilderten Ausführungsbeispiels abgewichen werden. So kann eine entsprechend verstärkte Ausführung der Ventillippen 13 den elastischen Bügel 14 und den federelastischen Ring überflüssig machen. Auch sind vorspringende Randleisten 15 am distalen Katheterende nicht zwingend notwendig, weil dieses Katheterende auch ohne Randleisten von außen tastbar ist. Anstelle des beschriebenen Lippenventils ist auch jedes andere Ventil einsetzbar, das, z.B. unter Federwirkung, normalerweise geschlossen ist und sich durch äußeren Druck öffnen lässt.

## Patentansprüche

1. Vorrichtung zur Behandlung der Harninkontinenz des Mannes, mit einem in die Harnröhre einführbaren Katheter (1), der an seinem proximalen Endabschnitt (11) einen ersten mit einem Fluid auffüllbaren Ballon (4) zur Abdichtung der Blase und zur Halterung des Katheters im Blasenlumen und distal von dem ersten Ballon (4) einen zweiten mit Fluid auffüllbaren Ballon (9) trägt, der im eingesetzten Zustand des Katheters außerhalb des Blasenschließmuskels (19) liegt, wobei beide Ballone (4, 9) durch längs der Katheterwandung verlaufende und am distalen Endabschnitt des Katheters verschlossene Kanäle (3, 7) mit dem Fluid auffüllbar bzw. entleerbar sind, und mit einem in dem distalen Endabschnitt des Katheters untergebrachten, selbsttätig schließenden Ventil (13), wobei die Länge des Katheters (1) derart bemessen ist, daß sein distales Ende im eingesetzten Zustand innerhalb der Harnröhre liegt und das Ventil (13) durch von außen auf die Harnröhre ausgeübten mechanischen Druck betätigbar ist,
dadurch gekennzeichnet,
daß der zweite Ballon (9) über einen Verbindungskanal (7) mit dem Inneren des ersten Ballons (4) kommuniziert, daß der Verbindungskanal (7) sich bis zum distalen Katheterende fortsetzt und seine distale Mündung (16) durch eine Membran verschlossen ist, daß die Länge des Katheters (1) derart bemessen ist, daß sein distales Ende im eingesetzten Zustand in der Harnröhre etwa beim distalen Ansatz des Skrotums (21) liegt, und daß mindestens eine der Öffnungen (6, 8), über welche der Verbindungskanal (7) in den ersten bzw. zweiten Ballon (4, 9) mündet, einen kleineren Querschnitt aufweist als die Öffnung (5), mit welcher der zu dem ersten Ballon (4) führende Kanal (3) in dessen Inneres mündet.

2. Vorrichtung nach Anspruch 1,
**dadurch gekennzeichnet**,
daß das Ventil (13) im distalen Endabschnitt des Katheters ein Lippenventil aus elastischem Material ist.

3. Vorrichtung nach Anspruch 2,
**dadurch gekennzeichnet**,
daß das Lippenventil in einer den Katheterquerschnitt verschließenden Membran ausgebildet ist.

4. Vorrichtung nach Anspruch 2 oder 3,
**dadurch gekennzeichnet**,
daß die Dichtränder des Lippenventils verdickt sind und/oder eine federelastische Verstärkung (14) enthalten.

5. Vorrichtung nach einem der Ansprüche 2 bis 4,
**dadurch gekennzeichnet,**
daß um die Dichtränder des Lippenventils ein federelastischer, ring- oder teilringförmiger Bügel (14) verläuft.

6. Vorrichtung nach Anspruch 5,
**dadurch gekennzeichnet**,
daß der Bügel in der Wand des distalen Katheterendes angeordnet ist.

7. Vorrichtung nach einem der Ansprüche 1 bis 6,
**dadurch gekennzeichnet,**
daß an der Außenwand des distalen Endabschnitts des Katheters (1) auf Höhe des Ventils (13) zwei einander diametral gegenüberliegende, von außen tastbare Vorsprünge (15) ausgebildet sind.

8. Vorrichtung nach Anspruch 7,
**dadurch gekennzeichnet**,
daß die Vorsprünge (15) am distalen Katheterende liegen und in mindestens einem davon der Kanal (3) und/oder der Verbindungskanal (7) mündet.

## Claims

1. Device for the treatment of male urinary incontinence with a catheter (1) that is introduced into the urethra and which carries a first balloon (4) towards its proximal end (11) that can be filled with fluid to seal the bladder and hold the catheter in the lumen of the bladder, and a second balloon (19) located distally from the first balloon (4) that can be filled with fluid which, with the catheter inserted, lies outside the vesical sphincter (19), whereby both balloons (4, 9) can be filled with and drained of the fluid by way of channels (3, 7) that run longitudinally along the catheter wall and are closed at the distal end of the catheter, and with an automatically closing valve (13) accommodated at the distal end of the catheter, whereby the length of the catheter (1) is such that, when inserted, its distal end lies inside the urethra and the valve (13) can be actuated by mechanical pressure acting on the urethra from the outside,
with the distinguishing feature
that the second balloon (9) communicates with the inside of the first balloon (4) by way of a connecting channel (7), that the connecting channel (7) continues to the distal end of the catheter and is closed by a membrane at its distal opening (16), that the length of the catheter (1) is such that, when inserted, its distal end lies inside the urethra more or less at the distal insertion of the scrotum (21), and that at least one of the openings (6, 8) by way of which the connecting channel (7) runs into the first and second balloons (4, 9) has a smaller cross section than the opening (5) by way of which the channel (3) that leads to the first balloon (4) runs into its inside.

2. Device according to claim 1,
with the distinguishing feature
that the valve (13) at the distal end of the catheter is a lip valve made of elastic material.

3. Device according to claim 2,
with the distinguishing feature
that the lip valve is formed in a membrane that seals the cross section of the catheter.

4. Device according to claim 2 or 3,
with the distinguishing feature
that the sealing edges of the seal valve are thickened and/or have a resilient elastic reinforcement (14).

5. Device according to one of the claims 2 to 4,
with the distinguishing feature
that a resilient elastic annular or partly annular hoop (14) runs round the sealing edges of the lip valve.

6. Device according to claim 5,
with the distinguishing feature
that the hoop is located in the wall of the distal catheter end.

7. Device according to one of the claims 1 to 6,
with the distinguishing feature
that two diametrically opposite and externally tangible projections (15) are formed in the outer wall of the distal end of the catheter (1) at about the level of the valve (13).

8. Device according to claim 7,
with the distinguishing feature
that the projections (15) are located at the distal end of the catheter and at least one of them forms the opening into which runs the channel (3) and/or the connecting channel (7).

## Revendications

1. Dispositif pour traiter l'incontinence urinaire masculine, muni d'un cathéter (1) pouvant être introduit dans l'urètre, qui porte à sa section d'extrémité (11) proximale un premier ballon (4) pouvant être rempli avec un fluide pour étancher la vessie et pour fixer le cathéter dans le calibre de la vessie et, distalement du premier ballon (4), un second ballon (9) pouvant être rempli avec du fluide, qui se trouve à l'extérieur du sphincter de la vessie (19) lorsque le cathéter est mis en place, les deux ballons (4, 9) pouvant être remplis ou vidés de fluide par les canaux fermés (3, 7) circulant le long de la paroi du cathéter et obturés à la section d'extrémité distale du cathéter, et muni d'une valve (13) à fermeture automatique logée dans la section d'extrémité distale du cathéter, la longueur du cathéter (1) étant conçue de telle sorte qu'étant mis en place, son extrémité distale se trouve à l'intérieur de l'urètre et que la valve (13) peut être actionnée par pression mécanique exercée de l'extérieur sur l'urètre, caractérisé en ce que le second ballon (9), communique avec l'intérieur du premier ballon (4) par un canal de raccordement (7), en ce que le canal de raccordement (7) se poursuit jusqu'à l'extrémité distale du cathéter et en ce que son embouchure distale (16) est obturée par une membrane, en ce que la longueur du cathéter (1) est conçue de telle sorte qu'étant mis en place, son extrémité distale se trouve dans l'urètre approximativement au niveau de la racine du scrotum (21), et,
en ce que au moins l'un des orifices (6, 8), par lesquels le canal de raccordement (7) débouche dans le premier ou le second ballon (4, 9), comporte une section transversale inférieure à celle de l'orifice (5), par lequel le canal (3) menant au premier ballon (4) débouche à l'intérieur de celui-ci.

2. Dispositif selon la revendication 1,
**caractérisé en ce que**,
la valve (13) à la section d'extrémité distale du cathéter est une valve à lèvre en matériau élastique.

3. Dispositif selon la revendication 2,
**caractérisé en ce que**,
la valve à lèvre est constituée dans l'une des membranes obturant la section transversale du cathéter.

4. Dispositif selon la revendication 2 ou 3,
**caractérisé en ce que**,
les bords d'étanchéité de la valve à lèvre sont épaissis et/ou contiennent un renfort (14) à élasticité de ressort.

5. Dispositif selon l'une des revendications 2 à 4,
**caractérisé en ce que**,
un étrier (14) circulaire ou partiellement circulaire à élasticité de ressort entoure les bords d'étanchéité de la valve à lèvre.

6. Dispositif selon la revendication 5,
**caractérisé en ce que**,
l'étrier est disposé dans la paroi de l'extrémité distale du cathéter.

7. Dispositif selon l'une des revendications 1 à 6,
**caractérisé en ce que**,
deux saillies (15) diamétralement opposées, palpables de l'extérieur, sont constituées à la paroi extérieure de la section d'extrémité distale du cathéter (1) à hauteur de la valve (13).

8. Dispositif selon la revendication 7,
**caractérisé en ce que**,
les saillies (15) se trouvent à l'extrémité distale du cathéter et en ce que le canal (3) et/ou le canal de raccordement (7) débouche dans au moins l'une de celles-ci.
